# EUROPEAN PATENT APPLICATION

(11) **EP 4 131 280 A1**
(43) Date of publication of application: **08.02.2023**
(21) Application number: 20951329.0
(22) Date of filing: 24.08.2020
(51) Int. Cl.: G16H 20/30

(54) **INFORMATION PROCESSING DEVICE, CONTROL METHOD, AND PROGRAM**

(71) Applicant: Japan Tobacco Inc., Tokyo 105-6927 (JP)
(72) Inventor: SHIMADA, Yurina, Tokyo 130-8603 (JP); KATO, Masato, Tokyo 130-8603 (JP)
(74) Representative: Hoffmann Eitle
(86) International application number: PCT/JP2020/031817
(87) International publication number: WO 2022/044073

(57) **Abstract**

In order to provide an arrangement with which it is possible to appropriately understand a rest time that a user is taking while using an inhalation device, an information processing device according to the present invention comprises a control unit that controls a process for calculating the user rest time on the basis of a start time of a first moving activity performed before an inhaling activity in which the user inhales an aerosol generated by the inhalation device, and an end time of a second moving activity performed after the inhaling activity.

## Description

### Technical Field

The present invention relates to an information processing device, a control method, and a program.

### Background Art

Inhaler devices, such as electronic cigarettes and nebulizers, generating material to be inhaled by users are in widespread use. For example, an inhaler device uses an aerosol source for generating an aerosol and a substrate including a flavor source for imparting a flavor component to the generated aerosol to generate the aerosol to which the flavor component is imparted. When a user inhales the aerosol that is generated by the inhaler device and to which the flavor component is imparted, the user can enjoy the flavor.

In recent years, techniques in which a communication function is provided in an inhaler device and various items of information are collected from the inhaler device to provide services have been studied. For example, Patent Literature 1 discloses a technique in which when a user uses an inhaler device to inhale an aerosol, biological information of the user is collected and their psychological stress is evaluated to recommend that the user should extend their break time.

### Citation List

### Patent Literature

Patent Literature 1: JP 2020-68739A

### Summary of Invention

### Technical Problem

However, with the technique disclosed in Patent Literature 1 described above, the break time is not necessarily grasped properly. To promote the user's physical and mental health, it is desirable to properly grasp how long the user actually takes a break.

Accordingly, the present invention has been made in view of the above-described issue, and an object of the present invention is to provide a system for allowing a proper grasp of the time period of a break taken while the user is using an inhaler device.

### Solution to Problem

In order to solve the above-described issue, according to an aspect of the present invention, there is provided an information processing device including: a controller that controls on the basis of a start time of a first movement action performed before an inhalation action in which a user inhales an aerosol generated by an inhaler device and an end time of a second movement action performed after the inhalation action, a process for calculating a break time period of the user.

The controller may assume the start time of the first movement action to be a start time of the break time period, assume the end time of the second movement action to be an end time of the break time period, and calculate the break time period.

The first movement action may be an action in which the user moves from a point that satisfies a predetermined condition as a start point, and the second movement action may be an action in which the user moves to a point that satisfies the predetermined condition as a destination point.

The controller may calculate as the break time period, a time period acquired by subtracting a time taken to move from the start point in the first movement action to the destination point in the second movement action from a time period from the start time of the first movement action to the end time of the second movement action.

When a difference between a time taken for a movement action performed after the inhalation action and a time taken for the first movement action is less than a first threshold, the controller may determine that the movement action is the second movement action.

When a time taken for a movement action performed after the inhalation action is greater than or equal to a second threshold, the controller may determine that the movement action is the second movement action.

The controller may determine a plurality of successive movement actions with a stop time having a length less than or equal to a third threshold therebetween to be one movement action.

The controller may control a process for notifying another user related to the user that the user starts the inhalation action.

The controller may control a process for notifying another user related to the user that the user starts a movement action that is assumed to be the first movement action.

The controller may control a process for providing to another user related to the user, positional information indicating a point at which the user performs the inhalation action.

The controller may control a process for providing to the user, information for encouraging the user to take a break, on the basis of a history of the break time period calculated previously.

In response to detection of a stress level of the user exceeding a predetermined threshold, the controller may control a process for providing to the user, information for encouraging the user to take a break.

The controller may control a process for providing to the user, information indicating the break time period.

The controller may control a process for providing to the user, information indicating a breakdown of the break time period including a time taken for the first movement action, a time taken for the second movement action, and a time taken for the inhalation action.

The controller may control a process for providing to the user, information indicating an amount of exercise in the first movement action and an amount of exercise in the second movement action.

In order to solve the above-described issue, according to another aspect of the present invention, there is provided a control method to be performed by an information processing device, the control method including: controlling on the basis of a start time of a first movement action performed before an inhalation action in which a user inhales an aerosol generated by an inhaler device and an end time of a second movement action performed after the inhalation action, a process for calculating a break time period of the user.

In order to solve the above-described issue, according to another aspect of the present invention, there is provided a program for causing a computer to function as: a controller that controls on the basis of a start time of a first movement action performed before an inhalation action in which a user inhales an aerosol generated by an inhaler device and an end time of a second movement action performed after the inhalation action, a process for calculating a break time period of the user.

### Advantageous Effects of Invention

As described above, according to the present invention, a system for allowing a proper grasp of the time period of a break taken while the user is using an inhaler device is provided.

### Brief Description of Drawings

[Fig. 1] Fig. 1 is a schematic diagram of an inhaler device according to a first configuration example.
[Fig. 2] Fig. 2 is a schematic diagram of an inhaler device according to a second configuration example.
[Fig. 3] Fig. 3 is a diagram of an example of the configuration of a system according to the present embodiment.
[Fig. 4] Fig. 4 is a diagram for explaining calculation of a break time period according to the present embodiment.
[Fig. 5] Fig. 5 is a flowchart of an example of the flow of a process performed in a user terminal according to the present embodiment.
[Fig. 6] Fig. 6 is a diagram for explaining calculation of a break time period according to a first modification.
[Fig. 7] Fig. 7 is a flowchart of an example of the flow of a process performed in the user terminal according to the first modification.

### Description of Embodiments

Hereinafter, a preferred embodiment of the present invention will be described in detail with reference to the attached drawings. Note that in the specification and drawings, structural elements having substantially the same functional configurations are assigned the same reference signs to thereby omit duplicated descriptions.

### 1. Configuration example of inhaler device

An inhaler device generates material to be inhaled by a user. In the example described below, the material generated by the inhaler device is an aerosol. Alternatively, the material generated by the inhaler device may be gas.

### (1) First configuration example

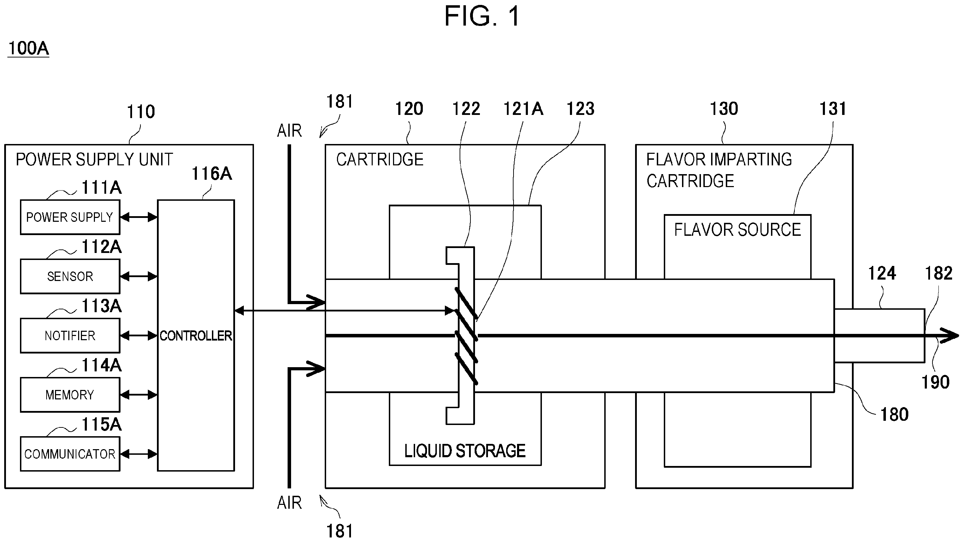

Fig. 1 is a schematic diagram of the inhaler device according to the first configuration example. As illustrated in Fig. 1, an inhaler device 100A according to the present configuration example includes a power supply unit 110, a cartridge 120, and a flavor imparting cartridge 130. The power supply unit 110 includes a power supply 111A, a sensor 112A, a notifier 113A, a memory 114A, a communicator 115A, and a controller 116A. The cartridge 120 includes a heater 121A, a liquid guide 122, and a liquid storage 123. The flavor imparting cartridge 130 includes a flavor source 131 and a mouthpiece 124. In the cartridge 120 and the flavor imparting cartridge 130, an airflow path 180 is defined.

The power supply 111A stores electric power. The power supply 111A supplies electric power to the structural elements of the inhaler device 100A under the control of the controller 116A. The power supply 111A may be a rechargeable battery such as a lithium ion secondary battery.

The sensor 112A acquires various items of information regarding the inhaler device 100A. In an example, the sensor 112A may be a pressure sensor such as a condenser microphone, a flow sensor, or a temperature sensor, and acquire a value generated in accordance with the user's inhalation. In another example, the sensor 112A may be an input device that receives information input by the user, such as a button or a switch.

The notifier 113A provides information to the user. The notifier 113A may be a light-emitting device that emits light, a display device that displays an image, a sound output device that outputs sound, or a vibration device that vibrates.

The memory 114A stores various items of information for operation of the inhaler device 100A. The memory 114A may be a non-volatile storage medium such as flash memory.

The communicator 115A is a communication interface capable of communication in conformity with any wired or wireless communication standard. Such a communication standard may be, for example, Wi-Fi (registered trademark) or Bluetooth (registered trademark).

The controller 116A functions as an arithmetic processing unit and a control circuit, and controls the overall operations of the inhaler device 100A in accordance with various programs. The controller 116A includes an electronic circuit such as a central processing unit (CPU) or a microprocessor, for example.

The liquid storage 123 stores an aerosol source. The aerosol source is atomized to generate an aerosol. The aerosol source is a liquid such as polyhydric alcohol or water. Examples of the polyhydric alcohol include glycerine and propylene glycol. The aerosol source may include a flavor component that is either derived from tobacco or not derived from tobacco. For the inhaler device 100A that is a medical inhaler such as a nebulizer, the aerosol source may include a medicine.

The liquid guide 122 guides, from the liquid storage 123, the aerosol source that is the liquid stored in the liquid storage 123, and holds the aerosol source. The liquid guide 122 is, for example, a wick formed by twining fiber material such as glass fiber or porous material such as porous ceramic. In this case, the capillary action of the wick guides the aerosol source stored in the liquid storage 123.

The heater 121A heats the aerosol source to atomize the aerosol source and generate the aerosol. In the example illustrated in Fig. 1, the heater 121A includes a coil wound around the liquid guide 122. When the heater 121A produces heat, the aerosol source held by the liquid guide 122 is heated and atomized to generate the aerosol. The heater 121A produces heat when receiving electric power from the power supply 111A. In an example, the electric power may be supplied in response to the sensor 112A detecting a start of the user's inhalation and/or an input of predetermined information. Subsequently, the supply of the electric power may be stopped in response to the sensor 112A detecting an end of the user's inhalation and/or an input of predetermined information.

The flavor source 131 is a structural element for imparting a flavor component to the aerosol. The flavor source 131 may include a flavor component that is either derived from tobacco or not derived from tobacco.

The airflow path 180 is a flow path of air to be inhaled by the user. The airflow path 180 has a tubular structure having an air inlet hole 181 and an air outlet hole 182 at both ends. The air inlet hole 181 is an inlet of air into the airflow path 180, and the air outlet hole 182 is an outlet of the air from the airflow path 180. The liquid guide 122 is on the airflow path 180 at an upstream position (closer to the air inlet hole 181), and the flavor source 131 is on the airflow path 180 at a downstream position (closer to the air outlet hole 182). Air flowing in through the air inlet hole 181 when the user inhales mixes with the aerosol generated by the heater 121A. Subsequently, as indicated by an arrow 190, the mixture fluid of the aerosol and the air passes through the flavor source 131 and is conveyed to the air outlet hole 182. When the mixture fluid of the aerosol and the air passes through the flavor source 131, the flavor component included in the flavor source 131 is imparted to the aerosol.

The mouthpiece 124 is to be held in a mouth of the user during inhalation. The mouthpiece 124 has the air outlet hole 182. When the user inhales with the mouthpiece 124 in his/her mouth, the mixture fluid of the aerosol and the air enters the oral cavity of the user.

The configuration example of the inhaler device 100A has been described above. The inhaler device 100A is not limited to the above configuration, and may be configured in various ways as exemplified below.

In an example, the inhaler device 100A does not have to include the flavor imparting cartridge 130. In this case, the cartridge 120 includes the mouthpiece 124.

In another example, the inhaler device 100A may include various types of aerosol sources. Still another type of aerosol may be generated by mixing a plurality of types of aerosols generated from the plurality of types of aerosol sources in the airflow path 180 and causing a chemical reaction.

In addition, means for atomizing the aerosol source is not limited to heating by the heater 121A. For example, the means for atomizing the aerosol source may be vibration atomization or induction heating.

### (2) Second configuration example

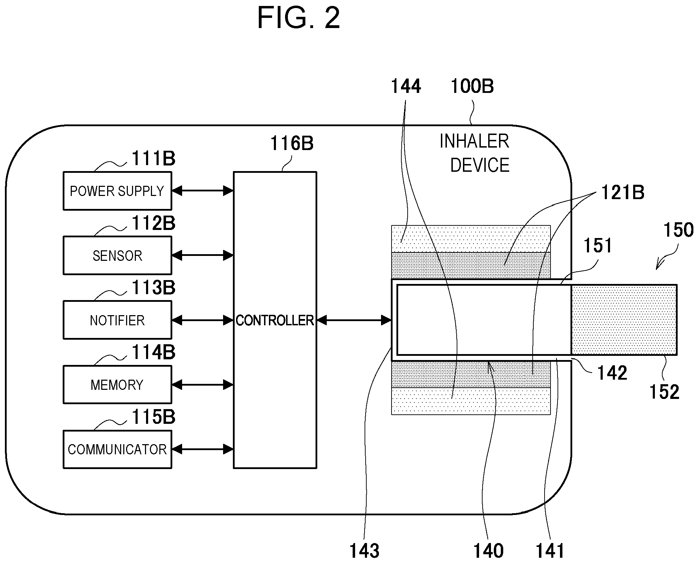

Fig. 2 is a schematic diagram of the inhaler device according to the second configuration example. As illustrated in Fig. 2, an inhaler device 100B according to the present configuration example includes a power supply 111B, a sensor 112B, a notifier 113B, a memory 114B, a communicator 115B, a controller 116B, a heater 121B, a holder 140, and a heat insulator 144.

The power supply 111B, the sensor 112B, the notifier 113B, the memory 114B, the communicator 115B, and the controller 116B are substantially the same as the respective corresponding structural elements included in the inhaler device 100A according to the first configuration example.

The holder 140 has an internal space 141, and holds a stick substrate 150 in a manner partially accommodated in the internal space 141. The holder 140 has an opening 142 that allows the internal space 141 to communicate with outside. The holder 140 holds the stick substrate 150 that is inserted into the internal space 141 through the opening 142. For example, the holder 140 may be a tubular body having the opening 142 and a bottom 143 on its ends, and may define the pillar-shaped internal space 141. The holder 140 also has a function of defining a flow path of air to be supplied to the stick substrate 150. An air inlet hole that is an inlet of air into the flow path is disposed, for example, on the bottom 143. An air outlet hole that is an outlet of the air from the flow path is the opening 142.

The stick substrate 150 includes a substrate 151 and an inhalation port 152. The substrate 151 includes an aerosol source. In the present configuration example, the aerosol source is not limited to a liquid and may be a solid. The stick substrate 150 held by the holder 140 includes the substrate 151 at least partially accommodated in the internal space 141 and the inhalation port 152 at least partially protruding from the opening 142. When the user inhales with the inhalation port 152 protruding from the opening 142 in his/her mouth, air flows into the internal space 141 through the air inlet hole (not illustrated), and the air and an aerosol generated from the substrate 151 reach inside the mouth of the user.

The heater 121B has a configuration the same as that of the heater 121A according to the first configuration example. However, in the example illustrated in Fig. 2, the heater 121B has a film-like shape and surrounds the outer circumference of the holder 140. Subsequently, heat produced from the heater 121B heats the substrate 151 of the stick substrate 150 from the outer circumference, generating the aerosol.

The heat insulator 144 prevents heat from transferring from the heater 121B to the other structural elements. For example, the heat insulator 144 may be a vacuum heat insulator or an aerogel heat insulator.

The configuration example of the inhaler device 100B has been described above. The inhaler device 100B is not limited to the above configuration, and may be configured in various ways as exemplified below.

In an example, the heater 121B may have a blade-like shape, and may be disposed so that the heater 121B protrudes from the bottom 143 of the holder 140 toward the internal space 141. In this case, the heater 121B having the blade-like shape is inserted into the substrate 151 of the stick substrate 150 and heats the substrate 151 of the stick substrate 150 from its inside. In another example, the heater 121B may be disposed so that the heater 121B covers the bottom 143 of the holder 140. In still another example, the heater 121B may be implemented as a combination of two or more selected from a first heater that covers the outer circumference of the holder 140, a second heater having the blade-like shape, and a third heater that covers the bottom 143 of the holder 140.

In another example, the holder 140 may include an opening/closing mechanism that at least partially opens and closes an outer shell defining the internal space 141. Examples of the opening/closing mechanism include a hinge. In addition, the holder 140 may sandwich the stick substrate 150 inserted into the internal space 141 by opening and closing the outer shell. In this case, the heater 121B may be at the sandwiching position of the holder 140 and may produce heat while pressing the stick substrate 150.

In addition, means for atomizing the aerosol source is not limited to heating by the heater 121B. For example, the means for atomizing the aerosol source may be induction heating.

In addition, the inhaler device 100B may also include the heater 121A, the liquid guide 122, the liquid storage 123, and the airflow path 180 according to the first configuration example. The air outlet hole 182 of the airflow path 180 may also serve as an air inlet hole to the internal space 141. In this case, a mixture fluid of the air and an aerosol generated by the heater 121A flows into the internal space 141, mixes further with an aerosol generated by the heater 121B, and then reaches the oral cavity of the user.

### 2. Configuration example of system

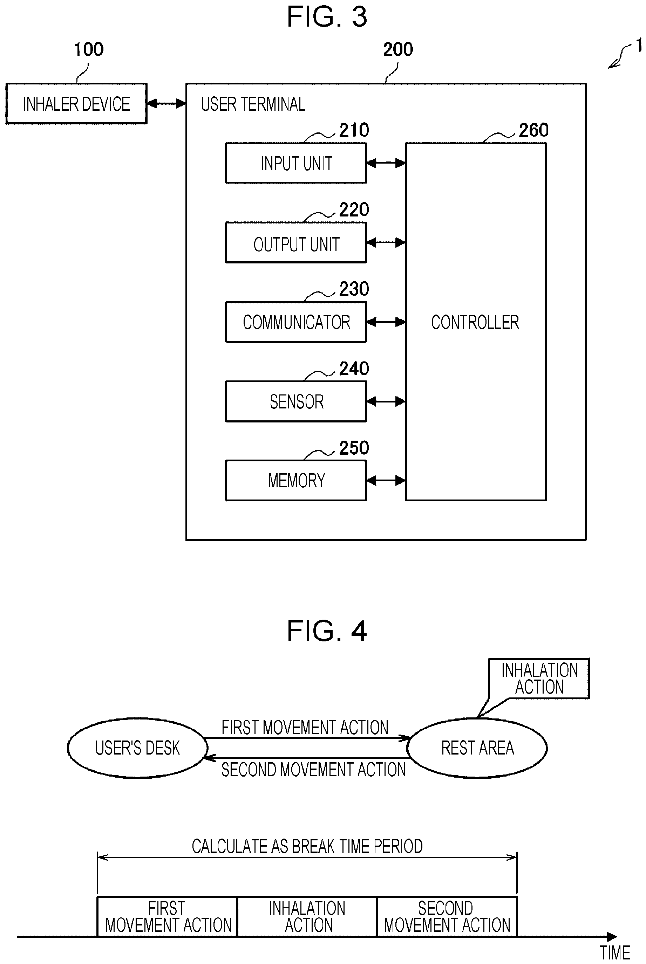

Fig. 3 is a block diagram of an example of the configuration of a system 1 according to the present embodiment. As illustrated in Fig. 3, the system 1 includes an inhaler device 100 and a user terminal 200. The inhaler device 100 and the user terminal 200 included in the system 1 are used by the same user.

### (1) Supplementary explanation about configuration of inhaler device 100

As described above, an inhaler device generates, for example, an aerosol as material to be inhaled by a user. The inhaler device 100 may have any of the first configuration example or the second configuration example described above. Inhalation, by a user, of an aerosol generated by an inhaler device is hereinafter also simply referred to as "inhalation" or "puffing". The action of inhalation by a user is hereinafter also referred to as an inhalation action.

The inhaler device 100 according to the present embodiment generates an aerosol to be inhaled by the user by using a substrate. A heater 121 is an example of a generator that generates an aerosol. The cartridge 120 and the flavor imparting cartridge 130 in the first configuration example and the stick substrate 150 in the second configuration example are examples of the substrate in the present invention. The inhaler device 100 generates an aerosol by using the substrate fitted to the inhaler device 100. In the first configuration example, the cartridge 120 and the flavor imparting cartridge 130 connected to the power supply unit 110 are examples of the substrate fitted to the inhaler device 100. In the second configuration example, the stick substrate 150 inserted into the inhaler device 100 is an example of the substrate fitted to the inhaler device 100.

### (2) Configuration of user terminal 200

The user terminal 200 is a terminal device carried and used by the user of the inhaler device 100. For example, the user terminal 200 is a smartphone or a tablet terminal. As illustrated in Fig. 3, the user terminal 200 includes an input unit 210, an output unit 220, a communicator 230, a sensor 240, a memory 250, and a controller 260.

The input unit 210 has a function of receiving input of various items of information. The input unit 210 may include an input device that receives input of information from the user. Examples of the input device include a button, a keyboard, a touch panel, and a microphone. In addition, the input unit 210 may include various sensors including an image sensor.

The output unit 220 has a function of outputting information. The output unit 220 may include an output device that outputs information to the user. Examples of the output device include a display device that displays information, a light-emitting device that emits light, a vibration device that vibrates, and a sound output device that outputs sound. The display device is, for example, a display. The light-emitting device is, for example, a light-emitting diode (LED). The vibration device is, for example, an eccentric motor. The sound output device is, for example, a speaker. The output unit 220 outputs information input from the controller 260 to thereby provide information to the user.

The communicator 230 is a communication interface for transmitting and receiving information between the user terminal 200 and another device. The communicator 230 performs communication in conformity with any wired or wireless communication standard. Such a communication standard may be, for example, a wireless local area network (LAN), a wired LAN, Wi-Fi (registered trademark), Near Field Communication (NFC), or Bluetooth (registered trademark).

The sensor 240 has a function of detecting various items of information. In an example, the sensor 240 may receive a Global Navigation Satellite System (GNSS) signal from a GNSS satellite (for example, a Global Positioning System (GPS) signal from a GPS satellite) and detect geographical positional information including the latitude, longitude, and altitude of the device. In another example, the sensor 240 may detect an acceleration. In another example, the sensor 240 may detect an angular velocity. In another example, the sensor 240 may detect biological information such as the body temperature and pulse of the user.

The memory 250 stores various items of information for operations of the user terminal 200. The memory 250 may be a non-volatile storage medium such as flash memory.

The controller 260 functions as an arithmetic processing unit and a control device and controls the overall operations of the user terminal 200 in accordance with various programs. The controller 260 includes, for example, an electronic circuit such as a central processing unit (CPU) or a microprocessor. In addition, the controller 260 may include a read-only memory (ROM) that stores, for example, a program and arithmetic parameters to be used and a random access memory (RAM) that temporarily stores, for example, parameters that change as appropriate. The user terminal 200 performs various processes under the control of the controller 260.

The functions of the controller 260 may be implemented as an application. This application may be pre-installed or downloaded. The functions of the controller 260 may be implemented as Progressive Web Apps (PWA).

### 3. Technical features

### 3.1 Basic process

### (1) Detection of inhalation action

The inhaler device 100 (specifically, a controller 116) functions as an inhalation action detector that controls a process for detecting an inhalation action by the user.

The inhaler device 100 detects an inhalation action by the user in response to a sensor 112 detecting a value generated in accordance with an inhalation action by the user. Examples of the value generated in accordance with an inhalation action by the user include the flow rate of air flowing through the airflow path 180, a pressure put on the airflow path 180, and a decrease in the temperature of the heater 121.

In addition, in response to the sensor 112 detecting a predetermined operation being performed, the inhaler device 100 may determine that an inhalation action by the user is performed. For example, the inhaler device 100 may detect a button operation for turning on the power of the inhaler device 100 as the start of an inhalation action and detect a button operation for turning off the power of the inhaler device 100 as the end of an inhalation action.

The inhaler device 100 transmits to the user terminal 200 information indicating that an inhalation action by the user is detected.

### (2) Detection of movement action

The user terminal 200 (specifically, the controller 260) functions as a movement action detector that controls a process for detecting a movement action by the user.

The user terminal 200 detects a movement action performed by the user in response to detection of information indicating that a movement action performed by the user is detected. In an example, the user terminal 200 may detect a movement action by the user on the basis of geographical positional information detected by the sensor 240. In another example, the user terminal 200 may count the number of steps on the basis of the acceleration and the angular velocity detected by the sensor 240 and detect a movement action by the user on the basis of the number of steps. In another example, the user terminal 200 may detect a movement action by the user on the basis of biological information (for example, a rise in the body temperature and in the pulse rate caused by walking) of the user detected by the sensor 240.

A movement action by the user is classified as a first movement action or a second movement action. The first movement action is a movement action performed before an inhalation action. The second movement action is a movement action performed after an inhalation action.

The first movement action may be an action in which the user moves from a point that satisfies a predetermined condition as the start point. The point that satisfies the predetermined condition is the user's place of work. The user's place of work is, for example, the user's desk in the office.

The first movement action may be an action in which the user moves to an inhalation-allowed point as the destination point. The inhalation-allowed point is a point at which an inhalation action by using the inhaler device 100 is allowed. The inhalation-allowed point is, for example, a rest area provided in the office as a place where the staff takes a break. The rest area is, for example, a smoking area equipped with a ventilation installation.

When the above-described two conditions are satisfied, the first movement action is an action in which the user moves from their place of work to the inhalation-allowed point. Based on these conditions, the time period of a break taken at the point away from the place of work can be calculated more accurately. As a matter of course, the user terminal 200 may assume a movement action performed before an inhalation action to be the first movement action regardless of the start point and the destination point.

The second movement action may be an action in which the user moves to the point that satisfies the above-described predetermined condition as the destination point. The second movement action may be an action in which the user moves from the inhalation-allowed point as the start point. When the above-described two conditions are satisfied, the second movement action is an action in which the user moves from the inhalation-allowed point to the user's place of work. Based on these conditions, the time period of a break taken at the point away from the place of work can be calculated more accurately. As a matter of course, the user terminal 200 may assume a movement action performed after an inhalation action to be the second movement action regardless of the start point and the destination point.

Note that the user's position may be detected on the basis of geographical positional information acquired by using GNSS. That is, whether the user's position is their place of work or the inhalation-allowed point may be determined on the basis of geographical positional information.

Alternatively, the user's position may be detected in response to the user terminal 200 receiving a signal transmitted from a communication device placed in a specific place. Examples of the specific place include the rest area, the place of work, and a movement pathway between the rest area and the place of work. For example, in response to the user terminal 200 receiving a signal transmitted from a communication device placed in the rest area, the user terminal 200 determines that the user is present in the rest area. The signal transmitted from the communication device is, for example, a beacon transmitted by using, for example, Bluetooth Low Energy (BLE (registered trademark)) or Low Power Wide Area (LPWA). The use of a beacon is effective specifically in indoors in which the accuracy of geographical positional information acquired by using GNSS decreases.

### (3) Calculation of break time period

The user terminal 200 (specifically, the controller 260) functions as a break-time calculator that controls a process for calculating the user's break time period.

The user terminal 200 calculates the user's break time period on the basis of the start time of the first movement action performed before an inhalation action and the end time of the second movement action performed after the inhalation action. The break time period in the present embodiment is a time spent taking a break while using the inhaler device 100. The break time period includes the time taken for the first movement action, the time taken for the second movement action, and the time taken for the inhalation action. The time taken for a movement action is also referred to as a moving time period. The time taken for an inhalation action is also referred to as an inhalation time period. With such a configuration, not only an inhalation time period but also moving time periods before and after the inhalation time period can be reckoned as the break time period. Taking into consideration that the user can refresh themselves also during a moving time period, with such a configuration, the user's break time period can be accurately grasped.

Fig. 4 is a diagram for explaining calculation of a break time period according to the present embodiment. As illustrated in the upper part of Fig. 4, the user performs the first movement action in which the user moves from their desk to the rest area, performs an inhalation action in the rest area, and performs the second movement action in which the user moves from the rest area to their desk. As illustrated in the lower part of Fig. 4, the user terminal 200 assumes the start time of the first movement action to be the start time of the break time period, assumes the end time of the second movement action to be the end time of the break time period, and calculates the break time period. With such a configuration, the entire period from the start time of the first movement action to the end time of the second movement action can be calculated as the break time period. In actuality, the user may take a break in the rest area in which, for example, the user talks with another user present at the time or may take a meal without performing an inhalation action. In this regard, with such a configuration, the time period during which the user is not actually performing an inhalation action but is taking a break can also be reckoned as the break time period. The user terminal 200 may calculate the time period from the end time of the first movement action to the start time of the second movement action as the inhalation time period.

Calculating the break time period includes calculating the length of the break time period. Calculating the break time period further includes identifying the start time of the break time period (that is, the start time of the first movement action) and identifying the end time of the break time period (that is, the end time of the second movement action). The user terminal 200 records information indicating the calculated break time period.

### (4) Providing information

The user terminal 200 (specifically, the controller 260) functions as a notification controller that controls a process for providing information to the user.

For example, the user terminal 200 provides information indicating the calculated user's break time period to the user. As the information indicating the break time period, information indicating the length of the break time period may be provided. As the information indicating the length of the break time period, the length of the break time period per break may be provided or the length of the total time period of breaks taken a plurality of times (for example, the total break time period in one month) may be provided. In addition, as the information indicating the break time period, at least one of the start time of the break time period (that is, the start time of the first movement action) or the end time of the break time period (that is, the end time of the second movement action) may be provided. With such a configuration, the user can easily grasp their break time period.

### (5) Flow of process

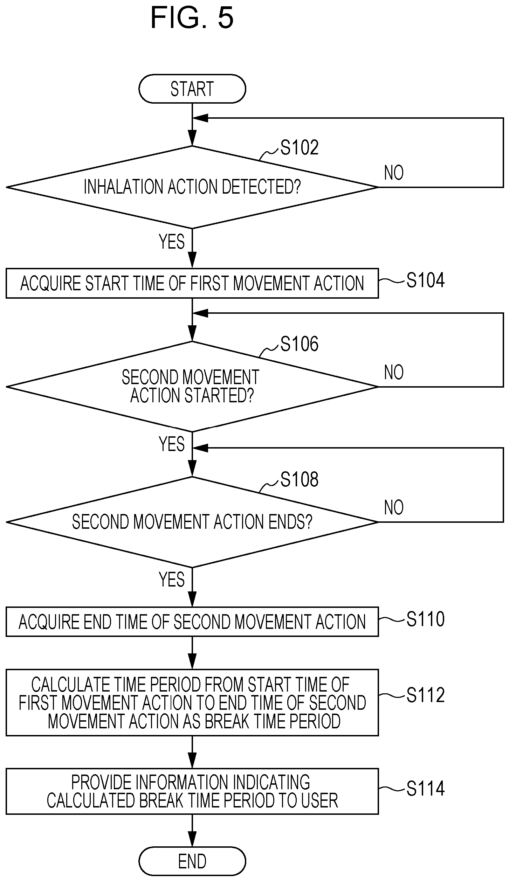

Fig. 5 is a flowchart of an example of the flow of a process performed in the user terminal 200 according to the present embodiment. It is assumed that the user terminal 200 continuously monitors information detected by, for example, the sensor 240 to detect a movement action.

As illustrated in Fig. 5, first, the user terminal 200 determines whether an inhalation action is detected (step S102). For example, the user terminal 200 determines whether information indicating that an inhalation action is detected is received from the inhaler device 100. If it is determined that an inhalation action is not detected (NO in step S 102), the user terminal 200 waits until an inhalation action is detected.

If it is determined that an inhalation action is detected (YES in step S102), the user terminal 200 acquires the start time of the first movement action performed before the inhalation action (step S104). For example, the user terminal 200 acquires the start time of a movement action performed before the inhalation action.

Next, the user terminal 200 determines whether the second movement action is started (step S106). For example, the user terminal 200 determines whether a movement action is started, on the basis of information detected by the sensor 240. If it is determined that the second movement action is not started (NO in step S106), the user terminal 200 waits until the second movement action is determined to be started.

If it is determined that the second movement action is started (YES in step S106), the user terminal 200 determines whether the second movement action ends (step S108). If it is determined that the second movement action does not end (NO in step S108), the user terminal 200 waits until the second movement action is determined to end.

If it is determined that the second movement action ends (YES in step S108), the user terminal 200 acquires the end time of the second movement action (step S110).

Next, the user terminal 200 calculates the time period from the start time of the first movement action to the end time of the second movement action as the break time period (step S112). The user terminal 200 provides information indicating the calculated break time period to the user (step S114).

### 3.2 Modifications

Various modifications described below may be combined as appropriate and implemented.

### (1) First modification

Although an example where the user's desk is assumed to be an example of the place of work and the user makes a round trip between their desk and the rest area has been described in the present embodiment described above, the present invention is not limited to such an example. The present invention is also applicable to a case where the user takes a break in the rest area during movement between two places of work.

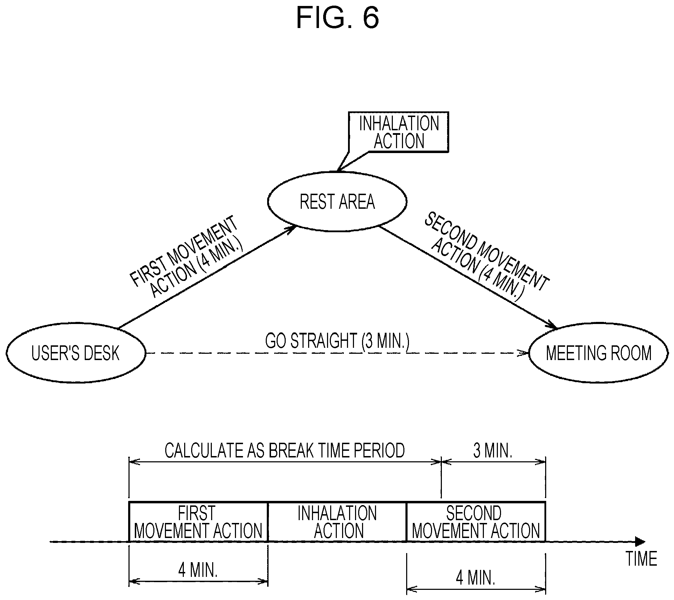

That is, a first place of work that is the start point in the first movement action may be different from a second place of work that is the destination point in the second movement action. The first place of work is, for example, the user's desk. The second place of work is, for example, a meeting room. Calculation of the break time period in such an example case will be described with reference to Fig. 6.

Fig. 6 is a diagram for explaining calculation of a break time period according to the first modification. As illustrated in the upper part of Fig. 6, the user performs the first movement action in which the user moves from their desk to the rest area, performs an inhalation action in the rest area, and performs the second movement action in which the user moves from the rest area to a meeting room. In this case, as illustrated in the lower part of Fig. 6, the user terminal 200 calculates as the break time period, a time period acquired by subtracting the time taken to move from the start point in the first movement action to the destination point in the second movement action from the time period from the start time of the first movement action to the end time of the second movement action. The time taken for the first movement action is four minutes, the time taken for the second movement action is four minutes, and the time taken to go straight to the rest area from the user's desk is supposed to be three minutes. Then, the user terminal 200 calculates as the break time period, a time period acquired by subtracting three minutes, which is the time taken to go straight to the rest area from the user's desk, from the time period from the time when the user starts moving from their desk to the time when the user arrives at the meeting room. The user terminal 200 may record as the end time of the break time period, a time that is before the end time of the second movement action by the time taken to go straight.

With the example described above, when the user takes a break during movement between two places of work, the user terminal 200 excludes from the break time period, the moving time taken to go straight without stopping by the rest area. This is because the moving time taken to go straight without stopping by the rest area is a time necessary for work. With such a configuration, the break time period from which the time necessary for work is excluded can be calculated.

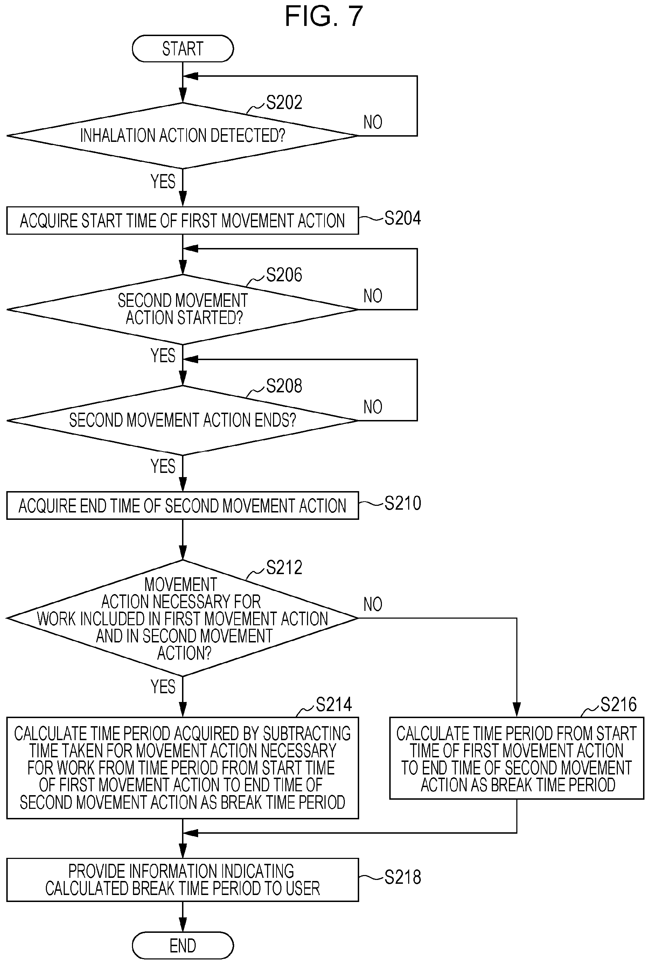

With reference to Fig. 7, the flow of a process in the present modification will be described.

Fig. 7 is a flowchart of an example of the flow of a process performed in the user terminal 200 according to the present modification. It is assumed that the user terminal 200 continuously monitors information detected by, for example, the sensor 240 to detect a movement action.

The process in steps S202 to S210 illustrated in Fig. 7 is the same as the process in steps S102 to S110 described above with reference to Fig. 5. Subsequently, the user terminal 200 determines whether a movement action necessary for work is included in the first movement action and in the second movement action (step S212). When the first movement action is a movement action in which the start point is the first place of work, the second movement action is a movement action in which the destination point is the second place of work, and the first place of work and the second place of work are different from each other, it is determined that a movement action necessary for work is included in the first movement action and in the second movement action.

If it is determined that a movement action necessary for work is included in the first movement action and in the second movement action (YES in step S212), the user terminal 200 calculates as the break time period, a time period acquired by subtracting the time taken for the movement action necessary for work from the time period from the start time of the first movement action to the end time of the second movement action (step S214). The time taken for the movement action necessary for work is the time taken to move from the start point in the first movement action to the destination point in the second movement action. If it is determined that a movement action necessary for work is not included in the first movement action or in the second movement action (NO in step S212), the user terminal 200 calculates the time period from the start time of the first movement action to the end time of the second movement action as the break time period (step S216). Subsequent to step S214 or S216, the user terminal 200 provides information indicating the calculated break time period to the user (step S218).

### (2) Second modification

When the rest area is crowded, the user may once leave the rest area in order to give space to others, and after a short movement action, return to the rest area to take a break. When such a short movement action is detected as the second movement action, accurate calculation of the break time period is difficult.

When the difference between the time taken for a movement action performed after an inhalation action and the time taken for the first movement action is less than a first threshold, the user terminal 200 may determine that the movement action is the second movement action. Under the assumption that the user makes a round trip between their desk and the rest area and takes a break, the time taken for the first movement action and the time taken for the second movement action can be considered to be substantially equal to each other. In this regard, with such a configuration, a short movement action that is performed in order to give space to others is not determined to be the second movement action, and therefore, the break time period can be accurately calculated. The first threshold may be a fixed value or may be increased or decreased in accordance with the time taken for the first movement action.

When the time taken for a movement action performed after an inhalation action is greater than or equal to a second threshold, the user terminal 200 may determine that the movement action is the second movement action. The second threshold is set on the basis of, for example, an expected time taken for a short movement action that is performed in order to give space to others. With such a configuration, a short movement action that is performed in order to give space to others is not determined to be the second movement action, and therefore, the break time period can be accurately calculated.

### (3) Third modification

During a movement action, the user may temporarily stop moving in order to, for example, wait for an elevator. When such a temporary stop is determined to be the start or end of the first movement action or the second movement action, accurate calculation of the break time period is difficult.

The user terminal 200 may determine a plurality of successive movement actions with a stop time having a length less than or equal to a third threshold therebetween to be one movement action. The stop time is a time in which the user does not move but stands still. The third threshold is set on the basis of a possible wait time during movement of the user between their desk and the rest area. With such a configuration, movement actions performed with a temporary stop for, for example, waiting for an elevator therebetween can be detected together as one first movement action or one second movement action, and therefore, the break time period can be accurately calculated.

### (4) Fourth modification

The user terminal 200 may give other users various notifications. In an example, giving other users a notification is transmitting information to terminal devices, such as smartphones, owned by other users. In another example, giving other users a notification is dispatching information to other users through a Social Networking Service (SNS).

The user terminal 200 may notify other users related to the user that the user starts an inhalation action. Other users related to the user are, for example, colleagues of the user who often take a break in the rest area together with the user. For example, the user terminal 200 registers in advance information about the smartphones of the colleagues as the destinations of notifications and provides at a timing when an inhalation action is detected, information indicating that the inhalation action is started. With such a configuration, a notification of the break timing can be automatically given to other users who want to take a break together with the user, without an operation by the user. The other users who receive such a notification can go to the rest area following the user and take a break together with the user.

The user terminal 200 may notify other users related to the user that the user starts a movement action that is assumed to be the first movement action. The first movement action is a movement action performed before an inhalation action, and therefore, the first movement action is not detected until an inhalation action is detected. Therefore, in response to detection of the start of a movement action, the user terminal 200 determines whether the movement action is assumed to be the first movement action. For example, the user terminal 200 calculates intervals, such as every day at around 10 a.m. or at intervals of three hours, at which the user takes a break, on the basis of a history of break time periods calculated previously. In response to detection of a movement action started at a timing corresponding to the intervals at which the user takes a break, the user terminal 200 determines that the movement action is the first movement action. In response to detection of the movement action that is assumed to be the first movement action, the user terminal 200 gives a notification to the smartphones of the colleagues. With such a configuration, a notification is given to other users at the timing when the user starts moving to the rest area, and therefore, the other users receiving the notification can go to the rest area together with the user to take a break together with the user.

The user terminal 200 may provide to other users related to the user, positional information indicating the point at which the user performs an inhalation action. Specifically, upon giving other users a notification described above, the user terminal 200 may also provide positional information indicating the point at which the user performs an inhalation action. In an example, in response to detection of an inhalation action started by the user, the user terminal 200 provides positional information of the rest area in which the user is actually present. In another example, in response to detection of a movement action that is started by the user and assumed to be the first movement action, the user terminal 200 provides positional information of a rest area to which the user often went previously at the same timing. With such a configuration, even when there are a plurality of rest areas, the user and other users can easily join together.

### (5) Fifth modification

The user terminal 200 may give the user further notifications.

The user terminal 200 may provide to the user, information for encouraging the user to take a break, on the basis of a history of break time periods calculated previously. For example, the user terminal 200 calculates intervals, such as every day at around 10 a.m. or at intervals of three hours, at which the user takes a break, on the basis of a history of break time periods calculated previously. When the start of a movement action is not detected even after a timing corresponding to the intervals at which the user takes a break, the user terminal 200 provides to the user, information for encouraging the user to take a break. With such a configuration, it is possible to encourage the user to periodically take a break, and this can promote the user's physical and mental health.

In response to detection of the user's stress level exceeding a predetermined threshold, the user terminal 200 may provide to the user, information for encouraging the user to take a break. For example, the user terminal 200 monitors biological information of the user to thereby calculate the user's stress level. The stress level may be calculated, for example, at a timing after the elapse of a predetermined time since the previous break taken by the user. When the calculated stress level exceeds the predetermined threshold, the user terminal 200 provides to the user, information for encouraging the user to take a break. With such a configuration, it is possible to encourage the user to appropriately take a break, and this can allow the user to refresh themselves and can increase the user's work efficiency.

The user terminal 200 may provide to the user, information indicating a breakdown of the break time period including the time taken for the first movement action, the time taken for the second movement action, and the time taken for the inhalation action. The time taken for the first movement action and the time taken for the second movement action may be provided such that the times are distinguishable from each other or such that the times are totaled. Information indicating a breakdown of the break time period per break may be provided, or information indicating a breakdown of the total break time period of a plurality of breaks (for example, the sum total of moving time periods and the sum total of inhalation time periods in one month) may be provided. With such a configuration, the length of a moving time period upon a break is visualized, and this can raise the user's awareness of health.

The user terminal 200 may provide to the user, information indicating the amount of exercise in the first movement action and that in the second movement action. In an example, when the user terminal 200 counts the number of steps during a movement action, the user terminal 200 may notify the user of the number of steps in the first movement action and that in the second movement action. In another example, the user terminal 200 may calculate calories burned during a movement action on the basis of the moving distance and the difference in level between before and after the movement and notify the user of calories burned during the first movement action and those burned during the second movement action. Information indicating the amount of exercise in the first movement action and the amount of exercise in the second movement action may be provided such that the amounts of exercise are distinguishable from each other or such that the amounts of exercise are totaled. Information indicating the amount of exercise per break may be provided, or information indicating the sum total of the amounts of exercise in a plurality of breaks (for example, the sum total of the amounts of exercise done in one month) may be provided. With such a configuration, the amount of exercise done in order to take a break is visualized, and this can raise the user's awareness of exercise and leads to a potential effect of encouraging the user to do more exercises.

### (6) Sixth modification

In another example, the rest area may be the place of work. In this case, when the end of an inhalation action is detected, and thereafter, neither the start of an inhalation action nor the second movement is detected within a predetermined time period, the user terminal 200 may record the timing when the end of the inhalation action is detected as the end time of the break time period. Accordingly, even when the user starts working in the rest area without the second movement, the break time period can be accurately calculated.

### 4. Supplementary description

Although a preferred embodiment of the present invention has been described in detail above with reference to the attached drawings, the present invention is not limited to the above-described example. It is obvious that one of ordinary skill in the art of the present invention can conceive of various changes or corrections without departing from the technical spirit stated in the claims, and it is understood that such changes and corrections also fall within the technical scope of the present invention as a matter of course.

For example, although an example where the user terminal 200 functions as the movement action detector, the break-time calculator, and the notification controller has been described in the above-described embodiment, the present invention is not limited to such an example. Any information processing device may function as the movement action detector, the break-time calculator, and the notification controller. Examples of the information processing device include the user terminal 200, the inhaler device 100, and a server on a network. The movement action detector, the break-time calculator, and the notification controller may be separately installed in two or more devices.

In an example, the movement action detector may be installed in a wearable device that is connected to the user terminal 200 so as to be able to communicate with the user terminal 200. In this case, the wearable device includes various sensors, detects a movement action by the user, and gives the user terminal 200 a notification.

In another example, the movement action detector may be installed in a plurality of devices. For example, a sensor for detecting geographical positional information may be installed in the user terminal 200, a sensor for detecting biological information may be installed in the inhaler device 100, and a sensor for detecting an acceleration and an angular velocity may be installed in the wearable device.

The processes described herein with reference to the flowcharts and sequence charts need not be performed in the illustrated order. Some process steps may be performed in parallel, an additional process step may be employed, and some process steps may be omitted.

Note that a series of processes performed by the devices described herein may be implemented as any of software, hardware, or a combination of software and hardware. Programs that constitute the software are stored in advance in, for example, a recording medium (non-transitory medium) that is included in each device or that is externally provided. Each program is loaded to a RAM upon execution by a computer and executed by a processor such as a CPU. Examples of the recording medium include a magnetic disc, an optical disc, a magneto-optical disc, and flash memory. In addition, the computer programs may be distributed via, for example, a network without using a recording medium.

Configurations as described below also fall within the technical scope of the present invention.
(1) An information processing device including:
   a controller that controls on the basis of a start time of a first movement action performed before an inhalation action in which a user inhales an aerosol generated by an inhaler device and an end time of a second movement action performed after the inhalation action, a process for calculating a break time period of the user.
(2) The information processing device according to (1) above, in which
   the controller assumes the start time of the first movement action to be a start time of the break time period, assumes the end time of the second movement action to be an end time of the break time period, and calculates the break time period.
(3) The information processing device according to (1) or (2) above, in which
   the first movement action is an action in which the user moves from a point that satisfies a predetermined condition as a start point, and
   the second movement action is an action in which the user moves to a point that satisfies the predetermined condition as a destination point.
(4) The information processing device according to (3) above, in which
   the controller calculates as the break time period, a time period acquired by subtracting a time taken to move from the start point in the first movement action to the destination point in the second movement action from a time period from the start time of the first movement action to the end time of the second movement action.
(5) The information processing device according to any one of (1) to (4) above, in which
   when a difference between a time taken for a movement action performed after the inhalation action and a time taken for the first movement action is less than a first threshold, the controller determines that the movement action is the second movement action.
(6) The information processing device according to any one of (1) to (5) above, in which
   when a time taken for a movement action performed after the inhalation action is greater than or equal to a second threshold, the controller determines that the movement action is the second movement action.
(7) The information processing device according to any one of (1) to (6) above, in which
   the controller determines a plurality of successive movement actions with a stop time having a length less than or equal to a third threshold therebetween to be one movement action.
(8) The information processing device according to any one of (1) to (7) above, in which
   the controller controls a process for notifying another user related to the user that the user starts the inhalation action.
(9) The information processing device according to any one of (1) to (8) above, in which
   the controller controls a process for notifying another user related to the user that the user starts a movement action that is assumed to be the first movement action.
(10) The information processing device according to any one of (1) to (9) above, in which
   the controller controls a process for providing to another user related to the user, positional information indicating a point at which the user performs the inhalation action.
(11) The information processing device according to any one of (1) to (10) above, in which
   the controller controls a process for providing to the user, information for encouraging the user to take a break, on the basis of a history of the break time period calculated previously.
(12) The information processing device according to any one of (1) to (11) above, in which
   in response to detection of a stress level of the user exceeding a predetermined threshold, the controller controls a process for providing to the user, information for encouraging the user to take a break.
(13) The information processing device according to any one of (1) to (12) above, in which
   the controller controls a process for providing to the user, information indicating the break time period.
(14) The information processing device according to any one of (1) to (13) above, in which
   the controller controls a process for providing to the user, information indicating a breakdown of the break time period including a time taken for the first movement action, a time taken for the second movement action, and a time taken for the inhalation action.
(15) The information processing device according to any one of (1) to (14) above, in which
   the controller controls a process for providing to the user, information indicating an amount of exercise in the first movement action and an amount of exercise in the second movement action.
(16) A control method to be performed by an information processing device, the control method including:
   controlling on the basis of a start time of a first movement action performed before an inhalation action in which a user inhales an aerosol generated by an inhaler device and an end time of a second movement action performed after the inhalation action, a process for calculating a break time period of the user.
(17) A program for causing a computer to function as:
   a controller that controls on the basis of a start time of a first movement action performed before an inhalation action in which a user inhales an aerosol generated by an inhaler device and an end time of a second movement action performed after the inhalation action, a process for calculating a break time period of the user.

### Reference Signs List

- 1: system
- 100: inhaler device
- 110: power supply unit
- 111: power supply
- 112: sensor
- 113: notifier
- 114: memory
- 115: communicator
- 116: controller
- 120: cartridge
- 121: heater
- 122: liquid guide
- 123: liquid storage
- 124: mouthpiece
- 130: flavor imparting cartridge
- 131: flavor source
- 140: holder
- 141: internal space
- 142: opening
- 143: bottom
- 144: heat insulator
- 150: stick substrate
- 151: substrate
- 152: inhalation port
- 180: airflow path
- 181: air inlet hole
- 182: air outlet hole
- 200: user terminal
- 210: input unit
- 220: output unit
- 230: communicator
- 240: sensor
- 250: memory
- 260: controller

## Claims

1. An information processing device comprising:
a controller that controls on the basis of a start time of a first movement action performed before an inhalation action in which a user inhales an aerosol generated by an inhaler device and an end time of a second movement action performed after the inhalation action, a process for calculating a break time period of the user.

2. The information processing device according to claim 1, wherein
the controller assumes the start time of the first movement action to be a start time of the break time period, assumes the end time of the second movement action to be an end time of the break time period, and calculates the break time period.

3. The information processing device according to claim 1 or 2, wherein
the first movement action is an action in which the user moves from a point that satisfies a predetermined condition as a start point, and
the second movement action is an action in which the user moves to a point that satisfies the predetermined condition as a destination point.

4. The information processing device according to claim 3, wherein
the controller calculates as the break time period, a time period acquired by subtracting a time taken to move from the start point in the first movement action to the destination point in the second movement action from a time period from the start time of the first movement action to the end time of the second movement action.

5. The information processing device according to any one of claims 1 to 4, wherein
when a difference between a time taken for a movement action performed after the inhalation action and a time taken for the first movement action is less than a first threshold, the controller determines that the movement action is the second movement action.

6. The information processing device according to any one of claims 1 to 5, wherein
when a time taken for a movement action performed after the inhalation action is greater than or equal to a second threshold, the controller determines that the movement action is the second movement action.

7. The information processing device according to any one of claims 1 to 6, wherein
the controller determines a plurality of successive movement actions with a stop time having a length less than or equal to a third threshold therebetween to be one movement action.

8. The information processing device according to any one of claims 1 to 7, wherein
the controller controls a process for notifying another user related to the user that the user starts the inhalation action.

9. The information processing device according to any one of claims 1 to 8, wherein
the controller controls a process for notifying another user related to the user that the user starts a movement action that is assumed to be the first movement action.

10. The information processing device according to any one of claims 1 to 9, wherein
the controller controls a process for providing to another user related to the user, positional information indicating a point at which the user performs the inhalation action.

11. The information processing device according to any one of claims 1 to 10, wherein
the controller controls a process for providing to the user, information for encouraging the user to take a break, on the basis of a history of the break time period calculated previously.

12. The information processing device according to any one of claims 1 to 11, wherein
in response to detection of a stress level of the user exceeding a predetermined threshold, the controller controls a process for providing to the user, information for encouraging the user to take a break.

13. The information processing device according to any one of claims 1 to 12, wherein
the controller controls a process for providing to the user, information indicating the break time period.

14. The information processing device according to any one of claims 1 to 13, wherein
the controller controls a process for providing to the user, information indicating a breakdown of the break time period including a time taken for the first movement action, a time taken for the second movement action, and a time taken for the inhalation action.

15. The information processing device according to any one of claims 1 to 14, wherein
the controller controls a process for providing to the user, information indicating an amount of exercise in the first movement action and an amount of exercise in the second movement action.

16. A control method to be performed by an information processing device, the control method comprising:
controlling on the basis of a start time of a first movement action performed before an inhalation action in which a user inhales an aerosol generated by an inhaler device and an end time of a second movement action performed after the inhalation action, a process for calculating a break time period of the user.

17. A program for causing a computer to function as:
a controller that controls on the basis of a start time of a first movement action performed before an inhalation action in which a user inhales an aerosol generated by an inhaler device and an end time of a second movement action performed after the inhalation action, a process for calculating a break time period of the user.
